# EUROPEAN PATENT APPLICATION

(11) **EP 1 787 576 A1**
(43) Date of publication of application: **23.05.2007**
(21) Application number: 05768645.3
(22) Date of filing: 02.08.2005
(51) Int. Cl.: A61B 1/04, H01L 21/56

(54) **RESIN COMPOSITION FOR MEDICAL EQUIPMENT SEALING AND MEDICAL EQUIPMENT FOR ENDOSCOPE HAVING BEEN SEALED THEREWITH**

(30) Priority: 03.08.2004 JP 2004226976
(71) Applicant: Olympus Corporation, Tokyo 151-0072 (JP)
(72) Inventor: KAGAWA, Ichiro Olympus IP Services Co.,Ltd., Tokyo 192-8512 (JP); MATSUMOTO, Jun Olympus IP Services Co., Ltd., Tokyo 192-8512 (JP)
(74) Representative: von Hellfeld, Axel
(86) International application number: PCT/JP2005/014121
(87) International publication number: WO 2006/013860

(57) **Abstract**

A sealing resin composition for sealing wiring portions of medical instrument, which comprises a thermosetting epoxy resin composition containing a fullerene compound.

## Description

### Technical Field

This invention relates to a resin composition for sealing medical instruments such as an endoscope, to a medical instrument such as an endoscope which is sealed with such a resin composition, and also to a method of manufacturing such a resin composition. In particular, this invention relates to a sealing resin composition to be employed for sealing the connecting portion of the terminal wiring of an image pickup device which is built in a distal end portion of endoscope.

### Background Art

Epoxy resin has been conventionally employed as a sealing resin for sealing the wiring of a substrate of a semiconductor device such as a semiconductor image pickup device. Further, it has been practiced to fill the epoxy resin with an inorganic filler such as silica-based compound for the purpose of enhancing the water resistance, heat resistance, moist heat resistance, water vapor permeability coefficient and adhesion of the epoxy resin as well as for the purpose of enhancing the stress relaxation and mold release characteristics of the epoxy resin.

Specifically, it has been proposed to fill the epoxy resin with an inorganic filler such as fumed silica, fused silica, crystalline silica, alumina, alumina hydrate, diatomaceous earth, mica, calcium carbonate, glass fiber, etc., at a ratio ranging from 5 to 1000% by weight (see for example, JP Patent Laid-open Publication (Kokai) No. 9-59385 (1997) and JP Patent Publication (Koukoku) No. 2-36148 (1990).

Since these sealing epoxy resins contain a large quantity of an inorganic filler, they are highly viscous or solid at ordinary temperature. Therefore, where a semiconductor device is sealed with a sealing resin, this highly viscous or solid epoxy resin composition is heated up to a molding temperature of not less than 200°C before the epoxy resin composition is subjected to transfer molding, injection molding or compression molding.

However, in the case where a connecting portion between the terminal wiring of an image pickup device to be built in an endoscope and the wiring of coaxial cable is to be sealed, to perform transfer molding, injection molding or compression molding of an epoxy resin composition containing a large quantity of an inorganic filler at a molding temperature of not less than 200°C is more likely to lead to the deformation or disconnection of wiring at the connecting portion and hence is considered difficult to performing the molding.

Further, it is also proposed to add a carbonaceous material such as carbon fiber, carbon or carbon nanotube to the epoxy resin composition. However, since these carbonaceous materials are inferior in electric insulating properties, it is impossible to employ them as a material for the sealing resin for sealing semiconductors or wiring portions.

It should be noted that it is also known to employ an epoxy resin composition excellent in moist heat resistance for sealing the connecting portion of a couple of constituent members of endoscope. More specifically, there has been proposed to employ an epoxy resin composition which has been proved excellent in moist heat resistance and in adhesive strength in the pressure cooker test (PCT) which was conducted under the conditions of a temperature of 135°C and 100%RH. (See, for example, JP-A 2003-126023.)

However, this epoxy resin composition is also not yet satisfactory with respect to water vapor permeability coefficient and is considered insufficient in reliability with respect to the moist heat resistance and adhesive strength if this epoxy resin composition is to be employed, as a sealing adhesive, for sealing the wiring portions of semiconductor devices or medical instruments.

An object of the present invention is to provide a resin composition for sealing medical instruments, which is excellent in water resistance, heat resistance and moist heat resistance, is very low in water vapor permeability coefficient, is capable of exhibiting a sufficient adhesion to a sealing portion, and is excellent in reliability and fabrication quality.

Another object of the present invention is to provide a medical instrument which is sealed by making use of the aforementioned resin composition.

A further object of the present invention is to provide a method of manufacturing the aforementioned resin composition for sealing medical instruments.

### Disclosure of Invention

According to one aspect of the present invention, there is provided a sealing resin composition for sealing wiring portions of medical instrument, which comprises a thermosetting epoxy resin composition containing a fullerene compound.

According to another aspect of the present invention, there is provided a method of manufacturing a sealing resin composition, which comprises dispersing a fullerene compound in a resin for forming a sealing resin composition to thereby obtain a mixture containing the resin and the fullerene compound.

According to a further aspect of the present invention, there is provided a medical instrument having an image pickup device built therein, wherein a connecting portion of terminal wirings of the image pickup device is sealed with the sealing resin composition as defined in above.

### Brief Description of Drawings

FIG. 1 is a cross-sectional view showing a distal end portion of the insert portion of endoscope, which was employed in one example of the present invention;
FIG. 2 is a cross-sectional view showing a mold for manufacturing a sample of a sealing compound for measuring moisture permeability coefficient; and
FIG. 3 is a cross-sectional view showing an apparatus for measuring moisture permeability coefficient.

### Best Mode for Carrying Out the Invention

The present inventors have made extensive studies on the water vapor permeability, moist heat resistance and adhesive strength of the epoxy resin composition to be employed as a sealing adhesive for sealing the wiring portions of medical instruments. As a result, the present inventors have found that, in the case where epoxy resin is mixed with an inorganic filler such as a silica compound, if the mixing ratio of the inorganic filler is increased, the viscosity of the resin mixture becomes higher, resulting in the deterioration of moldability and also the deterioration of water vapor permeability and also giving adverse influence to the adhesion of the resin mixture to a sealing portion and hence deteriorating the reliability of the sealed portion. Whereas, when fullerene compound is mixed with epoxy resin in place of the conventional inorganic filler, these problems can be overcome.

Especially, when the particle diameter of the fullerene compound is confined to a predetermined range, it is possible to obtain a thermosetting epoxy resin composition which is low in viscosity and in water vapor permeability.

Accordingly, the resin composition according to one aspect of the present invention, which is designed to be employed for sealing the wiring portions of a medical instrument, is characterized in that it comprises a thermosetting epoxy resin composition comprising a fullerene compound.

In the case of the resin composition according to one aspect of the present invention, since a fullerene compound is incorporated into epoxy resin, it is now possible to seal the wiring portion of medical instruments by making use of a sealing resin which is low in water vapor permeability under the conditions of a temperature of 135°C, a pressure of 3.2 atm and 100% wet heat environments and is excellent in reliability.

The sealing resin composition according to one aspect of the present invention is applicable to the sealing of the connecting portion of the terminal wirings of the image pickup device which is built in a distal end portion of endoscope, thus realizing excellent sealing.

Since the distal end portion of endoscope is designed to be introduced into a living body, a sterilization treatment is required to be performed on this distal end portion of endoscope, so that the sealed portion by making use of a sealing resin is required to be resistive to such a sterilization treatment. Especially, in the case of sterilization treatment in a water vapor atmosphere at high temperature and high pressure by means of autoclave, the sealed portion is required to be resistive to the permeation of water vapor.

Since the sealing resin composition according to one aspect of the present invention is very low in the permeation of water vapor even under high temperatures and high pressure, it is capable of exhibiting excellent properties against the permeation of water vapor even if it is subjected to a severe sterilization treatment.

As for the examples of fullerene compound which are useful in the sealing resin composition according to one aspect of the present invention, they include C₆₀ fullerene, C₇₀ fullerene, higher-order fullerene, fullerene derivatives, porous fullerene and frontier black. These fullerene compounds may be employed as a mixture of two or more kinds. It should be noted that "frontier black" means a residual matter that can be obtained by removing a mixture of fullerene from soot.

The mixing ratio of the fullerene compound should preferably be confined within the range of 50-70% by weight based on a total weight of the epoxy resin composition.

As for the fullerene compound, it should preferably be selected from those having a central particle diameter ranging from 0.01 to 3 µm. By making use of the fullerene compound whose central particle diameter is controlled in this manner, it is possible to obtain a sealing resin composition which is more suitable in viscosity and in moisture permeability coefficient.

Another aspect of the present invention is directed to a medical instrument having an image pickup device built therein, which is characterized in that a connecting portion of terminal wirings of the image pickup device is sealed by making use of the aforementioned sealing resin composition. This medical instrument is excellent in resistance to a sterilization treatment, in particular, to a treatment using high temperature and high pressure water vapor wherein an autoclave is employed.

The resin composition according to one aspect of the present invention can be manufactured as follows.

First of all, a fullerene compound is entirely charged into a planetary mixer (ACM-0.8LVT-C; Aikousha Seisakusho Co., Ltd.). Then, a portion of epoxy resin, i.e. 50 to 60% by weight, is added to the fullerene compound at a ratio of and then kneaded for 30 to 60 minutes to form a mixture. Subsequently, the rest portion of epoxy resin is added to the mixture and kneaded 30 to 60 minutes. As a result of these procedures, the fullerene compound is enabled to disperse in the epoxy resin as uniform particles each having a diameter ranging from 0.1 to 5 µm, thus obtaining a resin composition which is low in water vapor permeability and in viscosity.

It should be noted that the method of manufacturing the resin composition may not be limited to the aforementioned method. As for the planetary mixer, it is also possible to employ TK-Hibismix f model (Tokushu Kikai Kogyo Co., Ltd.).

Next, one embodiment of the present invention will be explained with reference to drawings.

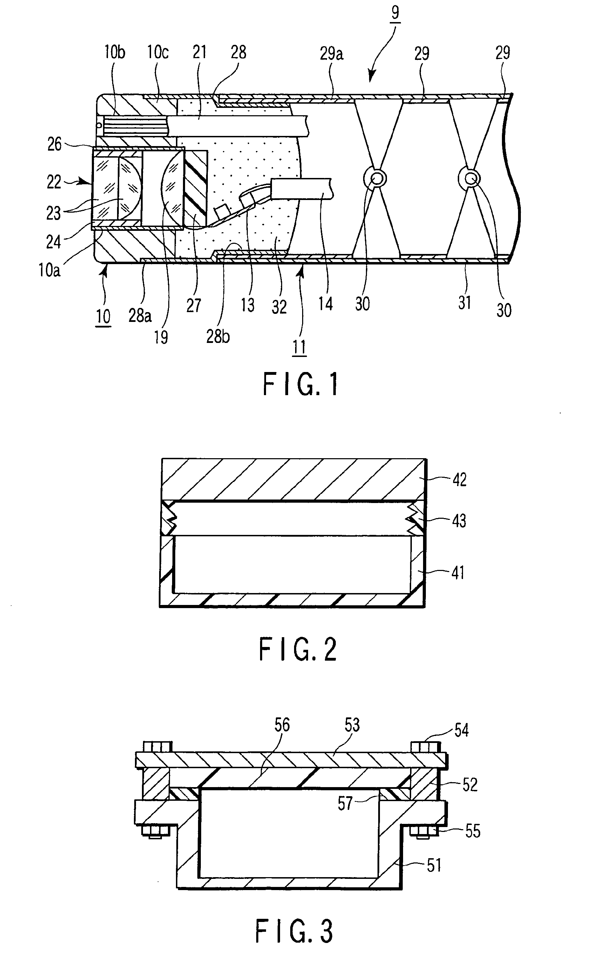

FIG. 1 is a cross-sectional view showing a distal end structure 10 of the insert portion 9 of electronic endoscope apparatus equipped with an electronic endoscope, representing one example of the present invention. Referring to FIG. 1, the distal end structure 10 is formed of a short cylindrical body which is provided with an image pickup unit-mounting hole 10a which is located at a lower central portion of the cylindrical body and extended in the axial direction of the cylindrical body, with a light guide-mounting hole 10b which is located at and piercing through an upper portion of the cylindrical body, with an air-blasting/water supply tube-mounting hole (not shown), with a forceps channel (not shown) for inserting a treating utensil such as forceps, and with a suction channel tube-mounting hole (not shown). A coupling portion 10c consisting of a step portion is formed on the outer circumferential surface of the proximal end portion of the distal end structure 10. To this coupling portion 10c is fixedly engaged with a large diametral portion 28a of the distal end portion of a connecting tube 28 formed of a connecting pipe.

The connecting tube 28 functions to connect the distal end structure 10 with a curved portion 11 to be disposed on the rear side of the distal end structure 10, so that the outer circumference of a fore-half portion of the connecting tube 28 is constituted by the large diametral portion 28a which is of the same diameter as a fore-half portion of the distal end structure 10 and a rear-half portion of the connecting tube 28 is constituted by a diametrally constricted portion 28b. This diametrally constricted portion 28b is fixedly engaged with an inner circumferential surface of the distal end portion of a curved piece 29a constituting a fore-most end portion of the curved portion 11. The outer circumferential surface of the curved portion 11 is covered with an outer covering 31 formed of a curved rubber. Accordingly, the outer diameter of the fore-half portion of distal end structure 10, the outer diameter of the fore-half portion of connecting tube 28, and the outer diameter of the outer covering 31 are all the same with each other.

The image pickup unit-mounting hole 10a is provided therein with an image pickup unit 22. This image pickup unit 22 is mainly constituted by a fixing barrel 26 for sustaining a lens frame 24 holding an objective optical system 23 comprising objective lens and also sustaining field lens 19 which is optically coaxially disposed behind the objective optical system 23, and by a charge coupled device (hereinafter referred to as a CCD) 27 which is disposed, as a solid state image pickup device, at an image-forming location behind the field lens 19. The fixing barrel 26 is designed to be fixedly fitted in the image pickup unit-mounting hole 10a, thus disposing the fixing barrel 26 inside the distal end structure 10.

The CCD 27 of the image pickup unit 22 is connected with a circuit board 13 mounted thereon with electronic components such as IC and capacitor. The circuit board 13 is connected with a signal cable 14 inserted into an insert portion. Accordingly, the CCD 27, the circuit board 13 and a distal end portion of the signal cable 14 are all designed to be disposed inside the connecting tube 28.

Further, a distal end portion of the light guide handle 21 that has been inserted into the insert portion is fixedly fitted in the light guide-mounting hole 10b of the distal end structure 10 in such a manner that the illuminating light emitted from the fore-end face of the light guide handle 21 and passing through a lighting window can irradiate a portion to be examined. Therefore, a portion of the light guide handle 21 which is extended rearward from the distal end structure 10 and located in the vicinity of the distal end structure 10 is also designed to be disposed inside the connecting tube 28. The interior of the connecting tube 28 is filled with a sealing material 32.

When the connecting tube 28 is filled with the sealing material 32, the components which are disposed inside the connecting tube 28 such as the CCD 27, the circuit board 13, a distal end portion of the signal cable 14, a distal end portion of the light guide handle 21, the air-blasting/water supply tube-mounting hole (not shown), and a distal end portion of the forceps channel (not shown) are all integrally fixed to the connecting tube 28.

It should be noted that in this embodiment, although the distal end structure 10 is constructed separate from the connecting tube 28, the present invention is not limited to such a structure. Namely, the distal end structure may be constructed such that a tubular portion corresponding to the connecting tube 28 is integrally extended from the rear end of the distal end structure 10.

As for the epoxy resin composition constituting the sealing material 32, it is possible to employ those having two or more epoxy groups per molecule. Specifically, it is possible to employ bisphenol A epoxy resin, bisphenol F epoxy resin, phenol novolac type epoxy resin, cresol novolac type epoxy resin, alicyclic epoxy resin, triazine nucleus-containing epoxy resin and a mixture of two or more kinds of these epoxy resins such as a modified resin.

As for the fullerene compound to be incorporated into the epoxy resin composition, it is possible to employ C₆₀ fullerene, C₇₀ fullerene, higher-order fullerene, fullerene derivatives, porous fullerene and frontier black. These fullerene compounds may be employed singly or in combination of two or more kinds. It is also possible to employ a mixture of these fullerene compounds. One example of such a mixture is a mixed fullerene (Frontier Carbon Co., Ltd.). This mixed fullerene comprises, as major components, C₆₀ fullerene and C₇₀ fullerene which constitute 85% of the entire components, the balance being higher-order fullerene.

The sealing epoxy resin composition according to one embodiment of the present invention may include a curing agent which is usually employed. Examples of such a curing agent are an acid anhydride such as phthalic anhydride, hexahyrophthalic anhydride, tetrahyrophthalic anhydride and pyromellitic anhydride; an amine compound such as ethylene diamine, diethylene diamine, triethylene tetramine, diethylaminopropyl amine, N-aminoethyl piperazine, bis(4-amino-3-methylcyclohexyl) methane, methane diamine, aliphatic amine, aromatic amine and aliphatic aromatic amine; a phenolic compound such as phenol, bisphenol A, bisphenol F, tetrabromobisphenol and cresol; and xylene resin.

In addition to these curing agents, the sealing epoxy resin composition may also contain a curing promoter which is usually employed, the examples of the curing promoter including, for example, tertiary amine such as BDMA, imidazoles and organophosphorus compound.

Further, the sealing epoxy resin composition according to one embodiment of the present invention may include various kinds of additives such as an inorganic filler, a silane coupling agent, a flame retardant, a colorant, a releasing agent, silicone oils, a low stress agent such as rubber.

Although the sealing epoxy resin composition of the present invention has been explained above with reference to one embodiment, the present invention should not be deemed as limited to the above embodiment. Accordingly, various changes and modifications may be made within the technical concept of the present invention. Before discussing the examples of the present invention, the method of evaluating the sealing epoxy resin composition will be explained as follows.

The measurement of adhesive strength was performed by making use of a test piece made of the same material as the constituent member of the aforementioned endoscope and on the basis of JISK 6850 "The method of testing the tensile shear adhesive strength of sealing compound". Further, the adhesive strength after the autoclave test was performed in such a manner that 600 groups of a pair of adhered test pieces were prepared and subjected to a sterilization treatment in a steam sterilization apparatus which is designed to perform the sterilization by making use of steam of 135°C, after which these test pieces were taken out of the apparatus and allowed to expose to air atmosphere and dried for 24 hours at ordinary temperature before measuring the adhesive strength.

It should be noted that the present inventors have devised, as explained below, a method of stably measuring the moisture permeability coefficient under the conditions of a temperature of 135°C and 100%RH (relative humidity).

Since the inner bottom face of mold is flat, it is possible to obtain a cured product having a substantially flat bottom on the occasion of molding a sample disc of sealing compound. However, it is difficult to obtain a cured product having a flat upper surface due to the shrinkage on curing. With a view to overcome this problem, the present inventors fabricated a structure wherein a fluorine plastic spacer 43 excellent in cushioning properties was interposed between a mold 41 and a cap 42 both made of PTFE (polytetrafluoroethylene) as shown in FIG. 2, thereby permitting the cap 42 to follow the shrinkage on curing of the sealing compound, thus enabling a redundant portion of resin to be discharged and making it possible to obtain a disc having a flat upper surface and a definite thickness.

A sample disc of sealing compound was manufactured by making use of a sample resin as described above and then set in place in an apparatus according to JISZ0208 as shown in FIG. 3. The apparatus shown in FIG. 3 is constructed such that an upper lid 53 is attached, through a spacer 52, to a cup 51 by making use of bolts 54 and nuts 55. the sample disc of sealing compound 56 is placed, through a packing 57, on the cup 51.

This apparatus differs greatly from the apparatus stipulated in JISZ0208 in the respect that the packing 57 made of special fluorine plastics was employed as a hermetic member.

By making use of this apparatus, the measurement of the moisture permeability coefficient was performed according to the pressure cooker test under the conditions of a temperature of 135°C and 100%RH. On this occasion, the visual examinations on the changes of the external appearance and on the cracking of the molded product as well as the measurement of volumetric specific resistance of the molded product after the test were performed.

10 parts by weight of aliphatic aromatic amine and a filler were added to 100 parts by weight of bisphenol A epoxy resin employed as a chief material, thus preparing 10 kinds of epoxy resin compositions, on which water resistance test and heat resistance test were performed. It should be noted that the details of these 10 kinds of epoxy resin compositions were as follows. Namely, they are a composition containing no filler (Comparative Example 1), a composition containing calcium carbonate (Maruo Calcium Co., Ltd.) (Comparative Examples 2-4), a composition containing fused silica for semiconductor (Denki Kagaku Co., Ltd.) (Comparative Examples 5-7), and a composition containing a mixed fullerene (Frontier Carbon Co., Ltd.) (Examples 1-3). The mixing ratio of the filler was set to 30 parts by weight, 60 parts by weight or 90 parts by weight.

The formulations of the composition and the results of experiment are shown in the following Table 1. In Table 1, "not studied" means that the test was not performed due to the phenomenon that due to a large quantity of the filler, the viscosity of the composition was caused to increase, generating a rugged surface of the molded product.

**Table 1**

| | Comp. Ex. 1 | Comp. Ex. 2 | Comp. Ex. 3 | Comp. Ex. 4 | Comp. Ex. 5 | Comp. Ex. 6 | Comp. Ex. 7 | Ex. 1 | Ex. 2 | Ex. 3 |
|---|---|---|---|---|---|---|---|---|---|---|
| Bisphenol A epoxy resin | 100 | 100 | 100 | 100 | 100 | 100 | 100 | 100 | 100 | 100 |
| Aliphatic aromatic amine | 10 | 10 | 10 | 10 | 10 | 10 | 10 | 10 | 10 | 10 |
| Calcium carbonate | | 30 | 60 | 90 | | | | | | |
| Fused silica for semiconductor | | | | | 30 | 60 | 90 | | | |
| Mixed fullerene | | | | | | | | 30 | 60 | 90 |
| Total | 110 | 140 | 170 | 200 | 140 | 170 | 200 | 140 | 170 | 200 |
| Viscosity (cps) | 8000 | 20000 | Solid-like | Solid-like | 10000 | 80000 | Solidified | 9000 | 20000 | 50000 |
| SUS+SUS Initial adhesive strength (MPa) | 35 | 40 | Not studied | Not studied | 45 | 45 | Not studied | 45 | 45 | 45 |
| SUS+SUS Water resistance adhesive strength (MPa) | 30 | 15 | Not studied | Not studied | 20 | 20 | Not studied | 30 | 35 | 35 |
| SUS+SUS Heat resistance adhesive strength (MPa) | 25 | 10 | Not studied | Not studied | 20 | 20 | Not studied | 45 | 45 | 45 |
| SUS+SUS Autoclave resistance (MPa) | 10 | 5 | Not studied | Not studied | 15 | 15 | Not studied | 20 | 25 | 27 |
| Moisture permeation coefficient (g·mm/m²day) | Fractured | Fractured | Not studied | Not studied | Fractured | Fractured | Not studied | 700 | 300 | 100 |

It will be recognized from Table 1 that the compositions of Examples 1-3 wherein a mixed fullerene was incorporated all exhibited excellent results in every tests on water resistance, heat resistance, autoclave resistance and the measurement of moisture permeability coefficient. Therefore, these compositions containing a mixed fullerene, which exhibited excellent results, were further formulated respectively to have a composition which was closer to one for actual loading and the examination was further performed on this composition.

Namely, 10 parts by weight of aliphatic aromatic amine was added to a chief material consisting of 70 parts by weight of bisphenol A epoxy resin and 30 parts by weight of fine particles of acrylic rubber, thus preparing a composition (Comparative Example 8), to which a mixed fullerene was further added at ratios of 30 parts by weight, 60 parts by weight or 90 parts by weight, respectively, thus preparing compositions (Examples 4-6), i.e. 4 kinds of epoxy resin compositions in total.

By making use of these four kinds of epoxy resin compositions, molded products (5 mm in thickness and 30 mm in diameter) were obtained by way of fabrication which was conducted for two hours at a temperature of 135°C. Then, these molded products were tested on the permeation of water vapor from the surface to the interior thereof and the measurement was performed on the quantity of permeated water vapor. Namely, by referring to JISZ0208 (Cup method), the moisture permeability coefficient of each of samples was measured in the environment of pressure cooker test (PCT) and under the conditions of a temperature of 135°C and 100%RH. Further, on this occasion, the visual examinations were made on the changes of the external appearance and on the cracking of the molded products.

**Table 2 Results of tests**

| | Comparative Example 8 | Example 4 | Example 5 | Example 6 |
|---|---|---|---|---|
| Bisphenol A epoxy resin | 70 | 70 | 70 | 70 |
| Fine particles of arylic rubber | 30 | 30 | 30 | 30 |
| Aliphatic aromatic amine | 10 | 10 | 10 | 10 |
| Mixed fullerene | | 30 | 60 | 90 |
| Total | 110 | 140 | 170 | 200 |
| Viscosity (cps) | 4000 | 8000 | 15000 | 40000 |
| SUS+SUS Initial adhesive strength (MPa) | 30 | 50 | 50 | 50 |
| SUS+SUS Autoclave resistance (MPa) | 25 | 30 | 35 | 35 |
| Steam permeation coefficient (g·mm/m².24h) | 500 | 250 | 200 | 150 |
| Evaluation of external appearance after autoclave resistance test | Cracked | No crack | No crack | No crack |

It was recognized from above Table 2 that the compositions of Examples 4-6 containing a mixed fullerene were all found excellent in initial adhesive strength and in anti-autoclave adhesive strength and low in moisture permeability coefficient and the cracking thereof was not recognized in the visual examinations. Whereas, the composition of Comparative Example 8 was found low in initial adhesive strength and in anti-autoclave adhesive strength and high in moisture permeability coefficient and moreover the cracking thereof was recognized in the visual examinations.

It should be noted that an anti-moisture sealing adhesive composition containing a mixed fullerene at a ratio of 50 parts by weight was coated on an image pickup device which was built in a distal end structure of endoscope and on the circuit board thereof, thereby confirming the workability thereof, and the mounting test thereof was performed. As a result, there was no problem with respect to all of the workability, the mounting test and anti-autoclave properties.

## Claims

1. A sealing resin composition for sealing wiring portions of medical instruments, which comprises a thermosetting epoxy resin composition containing a fullerene compound.

2. The sealing resin composition according to claim 1, wherein the wiring portions of medical instruments are a connecting portion of terminal wirings of an image pickup device which is built in a distal end portion of an endoscope.

3. The sealing resin composition according to claim 1, wherein the fullerene compound is at least one material selected from the group consisting of C₆₀ fullerene, C₇₀ fullerene, higher-order fullerene, fullerene derivatives, porous fullerene and frontier black.

4. The sealing resin composition according to claim 1, wherein the fullerene compound is contained in the resin composition at a ratio of 50-70% by weight based on a resin component in the resin composition.

5. The sealing resin composition according to claim 1, wherein the fullerene compound has a central particle diameter ranging from 0.01 to 3 µm.

6. The sealing resin composition according to claim 1, wherein the moisture permeability coefficient thereof in a saturated aqueous vapor atmosphere at a temperature of 135°C and a pressure of 3.2 atm is not more than 300 g·mm/m²·day.

7. A method of manufacturing a sealing resin composition, which comprises dispersing a fullerene compound in a resin for forming a sealing resin composition to thereby obtain a mixture containing the resin and the fullerene compound.

8. A medical instrument having an image pickup device built therein, wherein a connecting portion of terminal wirings of the image pickup device is sealed with the sealing resin composition as defined in claim 1.

9. The medical instrument according to claim 8, which is an endoscope device.
